## Europäisches Patentamt
## European Patent Office
## Office européen des brevets

(11) Numéro de publication: **0 046 704 B1**

# (12) FASCICULE DE BREVET EUROPEEN

(45) Date de publication du fascicule du brevet:
**24.10.84**

(21) Numéro de dépôt: **81401275.3**

(22) Date de dépôt: **07.08.81**

(51) Int. Cl.³: **G 01 B 5/00**, G 01 N 33/12,
A 61 B 5/10

(54) Appareil pour la mesure des caractéristiques de classification et pour la classification des animaux de boucherie abattus.

(30) Priorité: **14.08.80 FR 8017947**

(43) Date de publication de la demande:
**03.03.82 Bulletin 82/9**

(45) Mention de la délivrance du brevet:
**24.10.84 Bulletin 84/43**

(84) Etats contractants désignés:
**AT BE DE GB IT NL**

(56) Documents cités:
**CH - A - 353 488**
**FR - A - 2 039 434**
**FR - A - 2 070 280**
**FR - A - 2 462 205**

(73) Titulaire: **Augé, Jean, 1 rue François Sarre,
F-87350 Panazol (FR)**

(72) Inventeur: **Augé, Jean, 1 rue François Sarre,
F-87350 Panazol (FR)**

(74) Mandataire: **Chevallier, Robert Marie Georges, Cabinet
BOETTCHER 23, rue La Boétie, F-75008 Paris (FR)**

ACTORUM AG

## Description

L'invention concerne un appareil pour la mesure des caractéristiques de classification et la classification des animaux de boucherie abattus, dits carcasses, comprenant un châssis vertical de support pour des instruments de visée montés mobiles par rapport au châssis.

On connaît par FR-A-2 335 845 un appareil de mesure des distances entre les repères anatomiques de la carcasse d'un animal de boucherie. Un tel appareil permet de relever des caractéristiques dimensionnelles dans le plan de coupe d'une demi-carcasse, mais ne permet pas de relever d'autres caractéristiques importantes comme le rebondi ou l'épaisseur du rumsteack, l'épaisseur de renflement du tende de tranche, ou l'épaisseur musculaire du train de côte dans les carcasses de bovins, ou encore le développement externe des cuisseaux de veaux ou de gigots d'ovins, le développement musculaire de la longe (ou des reins) ou celui du carré (ou du dos). Il ne permet pas non plus de relever les caractéristiques volumétriques des carcasses entières d'ovins ou de veaux.

On connaît par ailleurs, par DE-A-2 602 524, un dispositif de mesure de demi-carcasses de porcs dans lequel des sondes mesurent le contour des jambons et l'épaisseur de lard, les valeurs de mesures étant introduites dans un calculateur programmé, déterminant une classe de qualité. Un tel appareil n'est adapté qu'à un type d'animal et la mise en place de la demicarcasse est cause de perte de temps.

On connaît aussi les brevets FR-A-2 070 280, 2 039 434 et CH-A-353 488 qui se rapportent à des moyens de mensuration du corps humain, pour en relever certaines dimensions ou pour en apprécier plus exactement certaines déformations comme des déviations vertébrales. Personne n'a jamais songé à les utiliser pour la classification des carcasses de boucherie, pour la raison évidente qu'ils sont appropriés à leur usage spécifique qui, en fait, n'implique pas une classification quelconque. Le mérite de l'invention, en comparaison de ces documents, est d'avoir compris qu'à partir d'éléments constructifs connus en soi, on pouvait en complément et en eméliorant les moyens décrits dans ces documents, parvenir à un appareil de classification de boucherie.

Le but de l'invention est donc de proposer un appareil universel, pouvant par simple échange d'instruments servir à classer aussi bien les demi-carcasses de bovins ou porcins que les carcasses d'ovins ou veaux.

Ce but est atteint selon l'invention grâce à l'appareil caractérisé dans la revendication 1 de la présente demande. On voit que grâce à l'invention, il est désormais possible de relever et d'enregistrer objectivement, non seulement des longueurs et largeurs de carcasses ou demi-carcasses, mais également d'autres caractéristiques telles que celles de la classification EUROPA définie en France par le décret 7 4 8o4 du 23.0.74 et l'arrêté du 14.5.75.

Pour faciliter les relevés des caractéristiques des demi-carcasses, à partir de trois plans de référence, dont le sol, il est conforme à l'invention que le châssis soit composite et comprenne au moins deux volets verticaux, mutuellement articulés, l'un étant fixe, l'autre mobile, chaque volet comprenant au moins une potence ou traverse horizontale règlable en hauteur.

Selon un mode de réalisation particulièrement simple, les moyens de saisie des données sont des moyens de mesure de déplacement des traverses ou potences, des chariots, de l'élongation et/ou de l'écartement des instruments de palpage, de l'écartement des instruments de visée.

Pour que l'appareil soit universel, il est conforme à l'invention que les instruments de palpage et/ou de visée soient montés amovibles sur les chariots porteurs d'instruments.

Il est avantageux que l'appareil comporte des moyens de positionnement de référence des carcasses ou demi-carcasses.

Selon un autre mode de réalisation avantageux, l'appareil comprend un calculateur programmé, des moyens de conversion analogique-numérique des données saisies à introduire dans le calculateur et des moyens d'introduction dans le calculateur de données complémentaires, telles que le poids et/ou autres indications introduites par un opérateur, comme le numéro de la carcasse.

Selon encore un autre mode de réalisation également avantageux, l'appareil comprend un dispositif d'affichage et/ou d'impression sur papier et/ou de marquage sur les carcasses de l'indice de classification déterminé par le calculateur.

En ce qui concerne les palpeurs, différents modes de montage sont possibles.

Cependant il est préférable que lesdits palpeurs soient montés sur des comparateurs tels que décrits dans les revendications dépendantes No. 7 à 11.

Pour la mesure du tende de tranche et du jambon, il sera conforme à l'invention qu'un comparateur comporte au moins deux palpeurs à élongation mesurable, respectivement un palpeur de positionnement sur un point défini, tel que l'os pubien, et au moins un palpeur à extrémité libre arquée reproduisant sensiblement le profil superficiel du tende de tranche.

Pour la mesure des caractéristiques des carcasses entières, il sera conforme à l'invention qu'un comparateur comprenne un premier palpeur télescopique de repérage de la séparation des noix, dont un élément porte une première règle s'étendant perpendiculairement, sur laquelle est monté coulissant un comparateur auxiliaire à trois éléments, dont deux symétriques et décalés selon la direction de la règle par rapport à l'élément médian, les éléments symétriques et l'élément médian constituant respectivement, l'un ou les uns, palpeur, l'autre ou les autres butées de comparaison, pour la mesure du développement du cuisseau ou gigot, l'autre élément du palpeur télescopique portant une seconde règle, s'étendant en sens opposé de la première règle, pour la mesure de la fente, par exemple grâce à un croisillon coulissant.

D'autres caractéristiques et avantages ressortiront le la description, qui sera donnée ci-après uniquement à titre d'exemple, d'un mode de réalisation de l'invention. On se reportera à cet effet aux dessins annexés, dans lesquels:

— la figure 1 est une vue d'ensemble en perspective schématique d'un appareil de l'invention dont on a retiré les instruments de palpage et/ou visée, figure dans laquelle le dispositif de positionnement des demi-carcasses est représenté en trait interrompu,

— la figure 2 est une vue schématique du dispositif de positionnement des demi-caracasses,

— la figure 3 illustre le mode de réalisation d'un palpeur,

— la figure 4 est une vue schématique de l'ensemble de calcul,

— les figures 5 et 6 sont des vues en perspective, à plus grande échelle qu'à la figure 1, des comparateurs pour les demi-carcasses,

— les figures 7a à 7e sont des dessins de carcasses servant à expliquer l'utilisation de l'appareil à la classification de carcasses, notamment de veaux, d'ovins, de porcs,

— les figures 8 et 9 sont des vues analogues aux figures 5 et 6 de comparateurs pour les carcasses examinées comme à la figure 7,

— la figure 10 est un marqueur pour l'appareil de l'invention.

L'ensemble décrit dans ce qui va suivre comprend un ensemble mécanique destiné à permettre la manoeuvre des instruments de palpage et/ou de visée, des éléments de captage destinés à mesurer les données, d'une unité de traitement des données et, éventuellement d'un mécanisme permettant de porter la classification sur chaque carcasse.

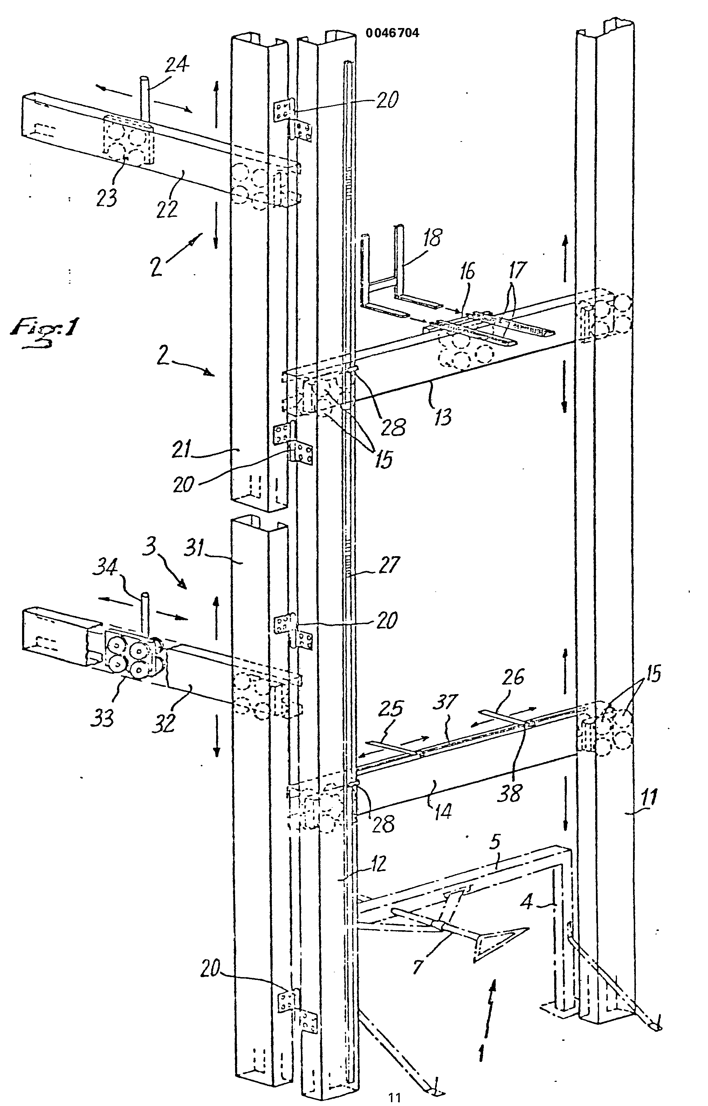

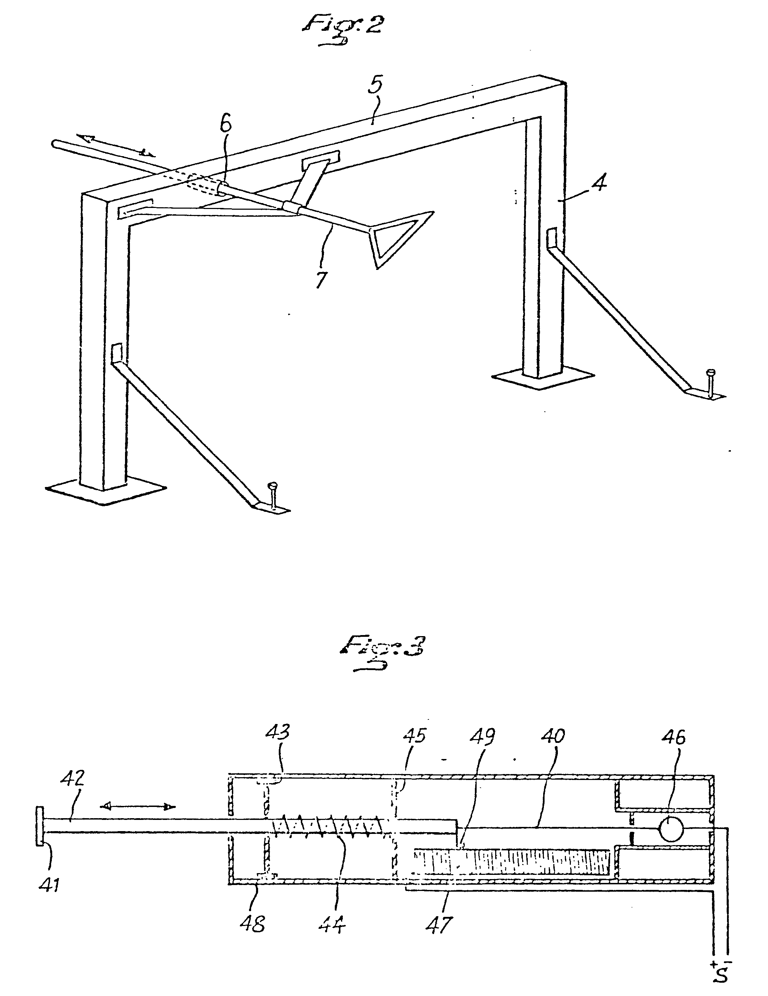

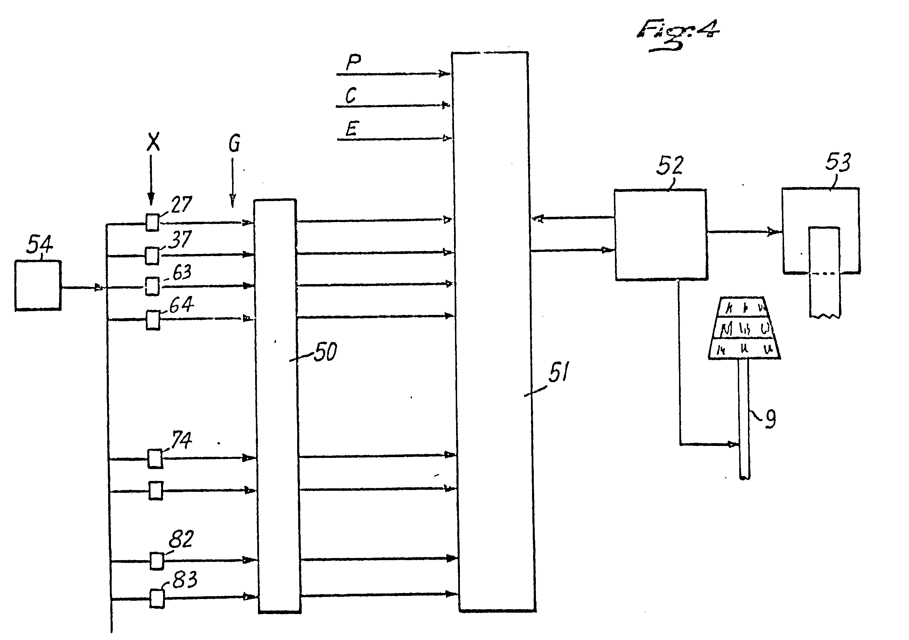

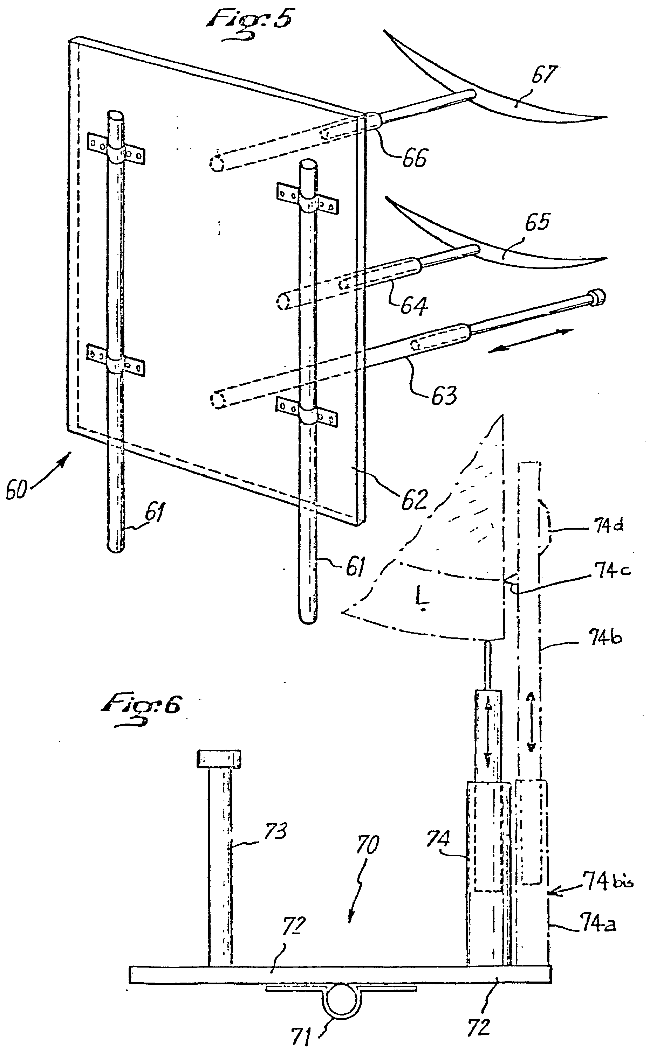

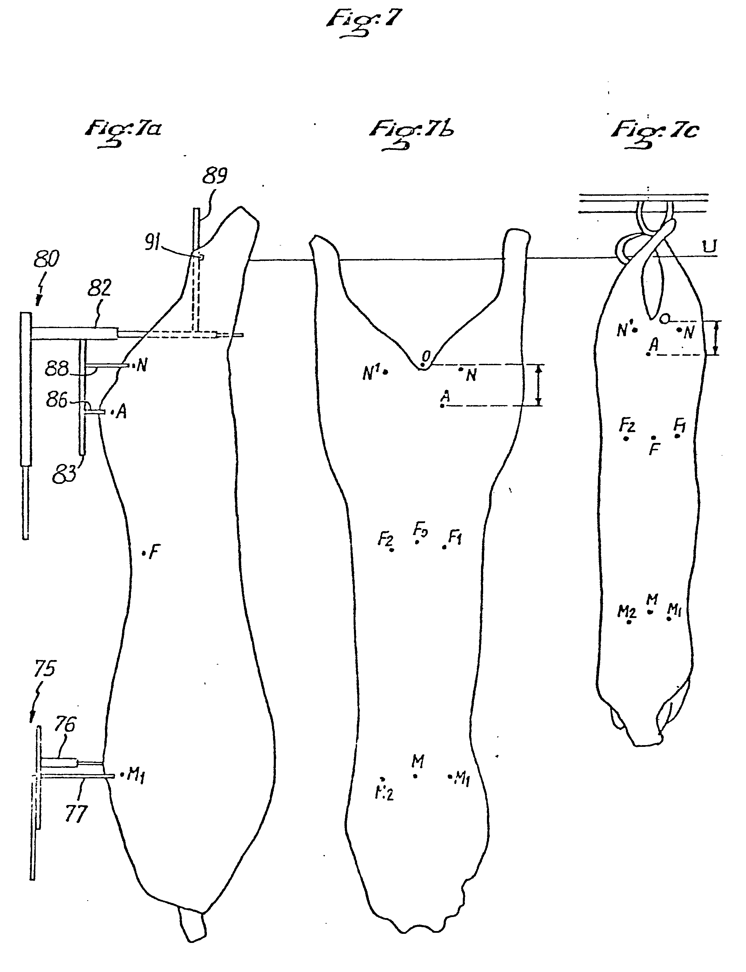

L'ensemble mécanique représenté à la figure 1 comprend deux volets, l'un fixe 1, l'autre pivotant 2. Le volet fixe 1 comprend deux montants verticaux 11, 12 écartés l'un de l'autre, constitués chacun par un profilé en U avec ailes de retour et deux traverses horizontales 13, 14 mobiles en hauteur grâce à des galets de guidage 15 pouvant rouler à l'intérieur des profilés en U constituant les montants 11, 12 servant de rails de guidage. Les traverses 13 et 14 sont donc règlables en hauteur et peuvent être immobilisées à hauteur voulue grâce à des contrepoids, non représentés. L'un des montants, 12, porte des demi-paumelles de paumelles 20 pour la fixation de deux volets pivotants superposés, respectivement 2 et 3, dont il sera parlé plus loin.

Les traverses horizontales 13, 14 mobiles en hauteur sont constituées, comme les montants 11, 12, par des profilés en U à ailes de retour, pouvant servir de rail de guidage à un chariot 16, monté, sur la figure 1, sur la traverse 13. Le chariot 16 porte deux glissières horizontales transversales 17 pour la fixation d'un instrument amovible, éventuellement par l'intermédiaire d'une équerre de fixation 18. Comme représenté à la figure 1, la traverse 14 peut recevoir deux curseurs 25, 26 pour deux instruments de visée optique.

Les volets mobiles 2 et 3 superposés sont substantiellement semblables et articulés chacun par deux paumelles 20 sur le montant 12 grâce auxquelles ils peuvent pivoter d'au moins 90° à partir du plan d'alignement avec le volet 1. Chacun des volets 2 et 3 comporte un montant vertical pivotant 21, 31 constitué par un profilé en U avec aile de retour et une potence 22, 32 mobile en hauteur grâce à des galets de guidage 15 pouvant rouler à l'intérieur des profilés en U constituant respectivement les montants 21, 31. Les potences 22, 32 sont donc règlables en hauteur et peuvent être immobilisées à hauteur voulue grâce à des contre-poids non représentés. Les potences 22, 32 sont également constituées par des profilés en U à ailes de retour, pouvant servir de guidage à un chariot 23, 33 portant un support 24, 34 pour un instrument de palpage et/ou de visée.

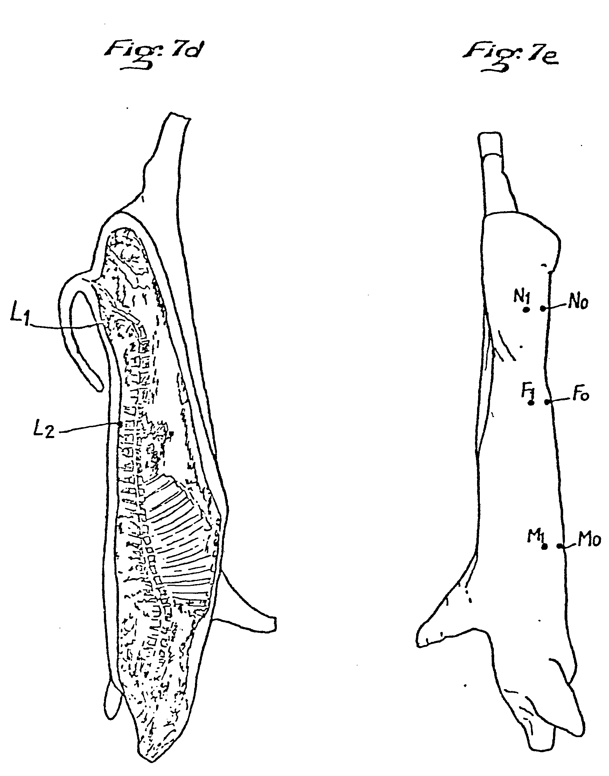

Par ailleurs, on installe au sol, légèrement en arrière du plan du volet 1 selon la figure 1, par exemple entre 0 et 20cm un portique 4 (voir figures 1 et 2) dont la poutre horizontale 5 est traversée dans un trou de guidage 6 par une barre escamotable 7 de positionnement de la carcasse. La hauteur des montants du volet fixe 1 approche à au moins 60cm celle des rails ou crochets de transfert. La hauteur du portique 4 est de l'ordre du tiers de celle des montants du volet 1.

Pour la mesure des déplacements relatifs des traverses ou potences par rapport aux montants, des chariots ou curseurs par rapport aux traverses ou potences, il est prévu des dispositifs potentiométriques. A cet effet les montants, ou au moins l'un d'eux, portent une règle potentiométrique linéaire 27 et les traverses ou potences portent un plot de contact 28. Pareillement les traverses ou potences peuvent porter des règles potentiométriques linéaires 37 et les curseurs portent un plot de contact 38, ainsi qu' éventuellement les chariots.

On voit, par ce qui a été décrit jusqu'ici que le volet fixe 1 réalise un système de mesure et de saisie de données à deux dimensions et que les palpeurs ajoutés aux chariots pourront permettre d'obtenir une troisième dimension. Il en est de même des volets mobiles 2 et 3.

On a vu qu'on peut monter sur chariots mobiles des palpeurs, qui seront décrits plus en détail plus loin. Ces palpeurs comportent un dispositif potentiométrique qu'on va maintenant décrire en regard de la figure 3. Ce dispositif comprend une butée 41 à l'extrémité d'une tige rigide 42 portant une butée 43 pour un ressort de compression 44 en appui contre une butée 45 du boîtier de guidage 48. La tige 42, guidée dans le boîtier 48 porte un plot de contact 49 pour un enroulement potentiométrique 47 raccordé à une borne d'une source de courant S, ledit plot de contact 49 étant, par ailleurs, raccordé à l'autre borne de la source de courant S par un fil 40 maintenu tendu par un enrouleur 46. On obtiendra de cette façon les mesures dans la troisième dimension, comme il a été annoncé précédemment. Toutes les mesures sont donc exprimées, grâce aux dispositifs potentiométriques, par des grandeurs électriques, telles que des tensions.

La figure 4 montre comment sont exploitées ces grandeurs et comment elles servent à établir la classification. Une alimentation 54 alimente les capteurs X constitués soit par les dispositifs potentiométriques 27, 37..., 83, 89....(dont il sera parlé plus loin), soit par les palpeurs 63, 64....., 74, 76......, 82, 86........, dont il sera également parlé plus loin, et qui sont conformes, dans leur partie électrique, à la description faite en regard de la figure 3. Les capteurs X délivrent alors un signal de grandeur G qui est dirigé sur un convertisseur analogique-numérique 50 qui les convertit en grandeur discrète introduite dans une mémoire 51, dans laquelle on introduit sous forme numérique d'autres données, telles que poids P, état d'engraissement E et couleur C. Une unité de calcul 52 est programmée pour traiter toutes les données, à commencer pour calculer les écarts entre points de mesures et pour en sortir une classification qui est imprimée, éventuellement avec d'autres caractéristiques partielles, sur une unité imprimante 53 et qui commande un marqueur 9 qui serait décrit plus en détail plus loin.

La différence de poids et de volume intratypes très variable chez les gros bovins reste assez limitée chez les autres types (ovins et veaux). Par ailleurs, en fin de chaîne d'abattage, la carcasse de gros bovin, ou de porcin se présente fendue par moitié suivant l'axe épineux, ce qui rend certaines régions anatomiques très accessibles. Or, les ovins et les veaux se présentent en carcasses entières, ce qui entrave l'accès des mêmes régions anatomiques. Enfin, la rapidité de classement sur chaîne d'abattage impose un choix de paramètre variable suivant les types. Par voie de conséquence, les comparateurs ou capteurs présenteront des variantes, tant dans leur disposition, leur écartement et leur forme mais restent constants dans leur mode de fonctionnement, dont on a déjà décrit l'essentiel, aussi bien en ce qui concerne le châssis que les palpeurs.

Des modes de réalisation particulièrement adaptés à divers cas seront maintenant décrits en même temps qu'on décrira le mode d'emploi de l'appareil pour ces divers cas.

1. Pour une demi-carcasse de boeuf, on retiendra sept données: a) le poids; b) le développement musculaire du tende de tranche (TT); c) le développement musculaire de rumsteack (R); d) le développement musculaire du faux-filet (FF); e) le développement musculaire du milieu de train de côtes (TC); f) la longueur ou distance entre 2 points pré-déterminés (L); g) la largeur ou distance entre 2 points pré-déterminés (1).

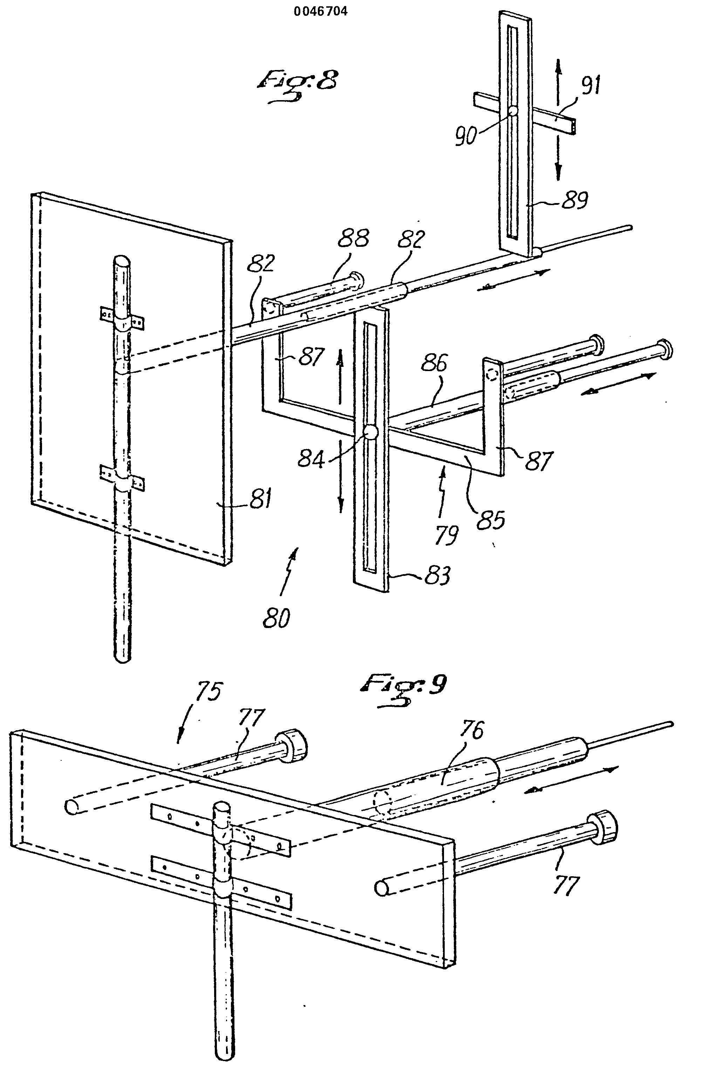

On place les deux volets 1 et 2 à 90° l'un de l'autre, comme représenté à la figure 1. On installe sur l'équerre 18 du chariot 16 un comparateur 60 (voir figure 5) par le moyen de deux tubes 61 de positionnement s'ajustant sur deux branches verticales de l'équerre 18. Les tubes 61 portent un plateau 62 disposé verticalement dans le plan de la traverse 13. Du plateau 62 s'étendent, vers l'intérieur du dièdre formé par les volets 1 et 2, trois palpeurs du type décrit, respectivement un palpeur 63 à bout normal, surmonté à environ 10 cm par un palpeur 64 à extrémité libre arquée horizontalement 65 et à 15 cm de celui-ci un autre palpeur 66 à extrémité libre arquée horizontalement 67. Les deux palpeurs en arc reproduisent sensiblement le profil superficiel du tende de tranche. Les dimensions précédentes ont été données pour des gros bovins. Elles pourront être modifiées pour chaque type de demi-carcasses, notamment pour les porcins.

Sur chacun des supports 24 et 34, on installe un autre comparateur 70 (voir figure 6) au moyen d'anneaux de guidage 71. Ce comparateur 70 comprend un plateau 72 installé dans le plan des potences 22 et 32 d'où s'étendent, vers l'intérieur du dièdre une butée fixe 73 et un palpeur 74.

D'autre part, on pousse la barre 7 en position de butée de la demi-carcasse. On présente alors celle-ci de telle manière que sa face dorsale vienne porter contre la barre 7 et que sa face interne vienne porter contre la poutre 5, les plans verticaux de la barre 7 et de la poutre 5 définissant ainsi des plans de référence.

Le poids est le poids fiscal obtenu par une bascule:

L'unité de calcul 52 recevra l'information P de la bascule, soit par connection calculateur-bascule, soit par entrée manuelle par un clavier.

Mesure du développement du tende de tranche (TT) et première partie de la mesure de longueur (L).

Par ses mouvements dans le plan du volet 1, le chariot 16 est positionné de telle manière que le palpeur 63 vienne porter sur l'os pubien. Le mouvement est prolongé jusqu'à ce que le palpeur 67 porte contre le muscle tende de tranche stoppant par contact le mouvement. L'enfoncement du palpeur 63 qui traduit le développement musculaire du tende de tranche par rapport à l'os pubien, est enregistré dans la mémoire.

Mesure du rumsteack (R).

Par les mouvements de la potence 22 et du chariot 23, celui-ci est positionné de telle manière que le palpeur 74, par pivotement sur les paumelles 20, vienne porter contre le sacrum. Le mouvement est prolongé jusqu'à ce que la butée 73 porte sur le rumsteack. L'enfoncement du palpeur 74, qui traduit le développement du rumsteack par rapport au sacrum est calculé et enregistré par la mémoire.

Mesure du faux-filet (FF)

Par les mouvements cités pour la mesure du rumsteack, le palpeur 74 du chariot 23 est amené à porter contre l'épine dorsale et enfoncé jusqu'à ce que la butée 73 porte sur le faux-filet. L'enfoncement du palpeur 74 qui traduit le développement du faux-filet par rapport aux vertèbres lombaires est calculé et enregistré par la mémoire.

Mesure du milieu de train de côtes (TC)

Toujours par les mouvements précités, le palpeur 74 du chariot 33 est amené à porter contre l'épine dorsale, au niveau prédéterminé, et enfoncé jusqu'à ce que la butée 73 porte sur le milieu de train de côtes. L'enfoncement du palpeur 74

qui traduit le développement du milieu de train de côtes par rapport aux vertèbres dorsales est calculé et enregistré par la mémoire.

Mesure de la largeur (1)

Sur le potentiomètre 37 de la traverse 14 circulent les deux curseurs 25 et 26, lesquels comportent chacun un système de visée type faisceau lumineux. Imposant des mouvements verticaux à la traverse 14, l'opérateur place la visée du curseur 25 à la jonction vertèbre 5è côte et celle du curseur 26 sur le bord interne du sternum. L'écartement des deux curseurs est calculé et enregistré comme donnée dans la mémoire.

Mesure de la longueur (L)

Sur le potentiomètre 27 du montant 12, circulent les deux curseurs 28, solidaires respectivement de la traverse 13 et de la traverse 14.

Dans les précédentes manoeuvres, l'opérateur a 1) placé la traverse 13 de façon que le palpeur 63 repose contre l'os pubien; 2) placé la traverse 14 de façon que la visée du curseur 25 soit sur la jonction 5è côte-vertèbre.

Par ces mesures potentiométriques, on connaîtra la distance séparant les deux curseurs 28 et cette mesure sera calculée et enregistrée dans la mémoire. Les données successivement calculées par l'unité de calcul sont traitées suivant une programmation permettant d'obtenir une note de conformation s'exprimant selon la classification officielle par les lettres E.U.R.O.P.A.

Le second critère retenu dans la classification officielle, c'est-à-dire, l'état d'engraissement (E), est relevé subjectivement et l'information s'exprimant en chiffres de 1 à 5, est introduite manuellement dans la mémoire et/ou l'unité de calcul.

Le marqueur 9, quatrième élément du dispositif et qui sera décrit plus loin, recevant par connection avec l'unité de calcul des impulsions électriques transformées par un moteur en mouvement rotatif, affichera le classement. Le manipulateur saisissant cet élément encrera les caractères affichés et frappant la carcasse, marquera sur cette dernière le classement affiché.

Le classement et le marquage étant effectués, l'opérateur fait pivoter le volet 2, tire sur la barre 7, ouvrant ainsi la voie pour que la carcasse soit poussée vers son lieu d'entreposage.

1. Carcasse ovin et veau

On a précédemment souligné la nécessité de retenir des paramètres différents selon le type de carcasse. Bien que la dénomination ne soit pas la même suivant qu'il s'agisse d'ovins ou de veaux, ce sont les mêmes régions anatomiques qui seront analysées.

On retiendra les paramètres suivants (voir figure 7)

a) Hauteur de la fente ou distance entre le point de séparation des noix 0 et le niveau d'accrochage U de la carcasse. Une distance importante indique un développement musculaire réduit.

b) Développement externe des cuisseaux (ou gigots) ou différence de profondeur entre l'anus A et deux points externes du cuisseau (ou gigot) N et N' points systématiquement prédéterminées par rapport à l'anus A. La différence de profondeur indique le développement musculaire.

c) Développement musculaire interne des cuisseaux (ou gigots) ou distance séparant l'anus A du point naturel de séparation des noix 0. Plus la distance est grande, plus le développement musculaire est important.

d) Développement musculaire de la longe (ou des reins) ou différence de profondeur entre un point lombaire de la colonne vertébrale, soit F0 et deux points F1 et F2 prédéterminés par le premier et symétriques par rapport à celui-ci.

e) Développement musculaire du carré (ou du dos) ou différence de profondeur entre un point dorsal de la colonne vertébrale M0 et deux points M1 et M2 prédéterminés par le premier et symétriques par rapport à celui-ci.

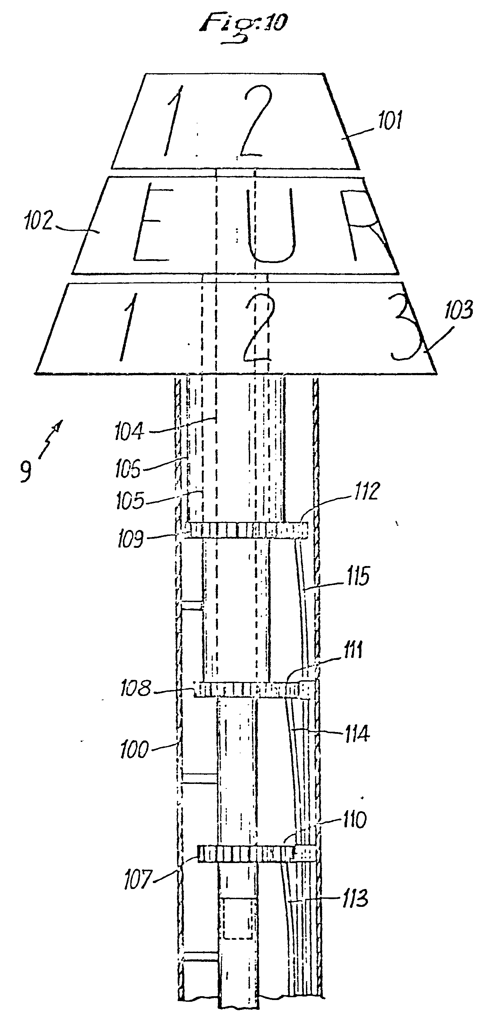

Pour procéder à ces mesures (voir figures 7a et 7b pour les veaux, 7c pour les ovins), on installe sur le chariot 23 un comparateur 80 (figure 8) et sur le chariot 33 un comparateur 75 (figure 9). L'un comme l'autre ont leurs organes de palpage dirigés vers l'intérieur du dièdre qui est règlé à angle droit, le volet 1 ne servant alors que de support au volet 2.

Le comparateur 80 porte une plaque de référence 81 disposée verticalement dans le plan de la potence 22. Un palpeur télescopique potentiométrique 82 porte sur sa branche fixe une règle 83 potentiométrique, s'étendant vers le bas avec un curseur à contact 84 portant un comparateur auxiliaire 79 constitué par une branche horizontale transversale 85 règlable en hauteur solidaire d'un second palpeur téléscopique potentiométrique 86 parallèle au palpeur 82. La branche horizontale transversale 85 se termine par deux bras verticaux 87 portant chacun une butée 88 dirigée dans le même sens que les plapeurs. La partie mobile du palpeur 82 porte une règle potentiométrique 89 s'étendant vers le haut avec un curseur à contact 90 portant une branche horizontale transversale ou croisillon 91 règlable en hauteur.

Le comparateur 75 comporte un palpeur téléscopique 76, en position légèrement inférieure par deux butées 77 s'étendant parallèlement.

Mesure du développement externe du cuisseau (ou gigot)

Par les mouvements verticaux et pivotants du volet 2 et par déplacement du chariot 23 celui-ci est positionné de telle manière que le palpeur 82 vienne effleurer la séparation des noix 0. Le curseur et les palpeurs qui lui sont solidaires sont déplacés verticalement jusqu'à ce que le palpeur 86 soit à hauteur de l'orifice anal. Le mouvement de pivotement du volet 2 est prolongé jusqu'à ce que les butées 88 portent contre le muscle du cuisseau (points N et N').

L'enfoncement du palpeur 86 opéré par butée traduit le développement externe du cuisseau ou gigot, est par le moyen cité précédemment enregistré, par l'unité de calcul.

Mesure de la fente

La règle 89, qui dans la première manoeuvre joue le rôle de butée, constitue également le boîtier qui contient, excepté le croisillon coulissant 91, tous les éléments du palpeur. L'opérateur positionne le croisillon 91 à hauteur du support ou crochet de la carcasse. Par le curseur 90, l'unité de calcul enregistre les informations recueillies, soit la distance séparant le support U du point 0 de la carcasse.

Mesure du développement interne du cuisseau ou gigot

La règle 83 constitue tout comme la règle 89 un boîtier contenant également, excepté le palpeur 86, tous les éléments du palpeur. L'opérateur positionne le palpeur 86 à la hauteur anale A. Toujours par le même procédé, l'unité de calcul enregistre les informations recueillies.

Mesure du développement musculaire de la longe ou des reins

Par des mouvements verticaux et pivotants du volet 2 et par déplacement du chariot 33, celui-ci est positionné de telle manière que le palpeur 76 porte contre l'épine dorsale au niveau lombaire F0. Le mouvement de pivotement du volet 2 est prolongé jusqu'à ce que les butées 77 portent sur le muscle, F1 et F2 (voir figures 7b et 7c).

L'enfoncement du palpeur 76 qui traduit le développement musculaire de la longe ou reins par rapport à l'épine dorsale est enregistré suivant les moyens précités par l'unité de calcul.

Mesure du développement musculaire du carré

Par les mêmes mouvements que précédemment le chariot 33 est positionné de telle manière que le palpeur 76 porte contre l'épine dorsale au niveau dorsal M0. Le mouvement de pivotement est prolongé jusqu'à ce que les butées 77 portent le muscle en M1 et M2.

L'enfoncement du palpeur 76 qui traduit le développement musculaire du carré par rapport à l'épine dorsale est enregistré suivant le moyen cité par l'unité de calcul.

Les données successivement enregistrées par l'unité de calcul sont traitées suivant une programmation permettant d'obtenir une note de conformation s'exprimant selon la classification officielle par les lettres E.U.R.O.P.A.

Le ou les autres critères retenus officiellement sont relevés subjectivement et l'information est introduite manuellement dans la calculatrice.

Tout comme pour le boeuf, le quatrième élément du dispositif recevra et affichera les mêmes conformations. Le manipulateur marquera la carcasse ainsi classée.

On décrira maintenant en regard de la figure 10, un marqueur 9 par encrage destiné à marquer les carcasses en fonction de la classification déterminée par l'unité de calcul.

Trois couronnes annulaires tronconiques 101, 102, 103 sont disposées les unes au-dessus des autres pour constituer un ensemble tronconique. Chaque couronne porte ou des chiffres ou des lettres marqueuses, par exemple de 1 à 5 ou les lettres E.U.R.O.P.A. Elles sont montées coaxialement et peuvent tourner indépendamment les unes des autres par entaînement par trois arbres coaxiaux, respectivement 104, 105, 106 à la base de chacun desquels se trove une couronne dentée, respectivement 107, 108, 109. A chaque couronne dentée est associée une roue dentée motrice 110, 111, 112, entraînée par un câble 113, 114, 115 sous gaine chacun entraîné par un moteur, par exemple du type pas-à-pas commandé lui-même par l'unité de calcul en concordance avec l'impression par l'unité d'impression 53. L'ensemble des arbres coaxiaux, des roues dentées et des câbles sous gaine est contenu dans un boîtier 100 d'où émergent les couronnes 101 à 103 servant à marquer directement les carcasses par une fenêtre prévue à cet effet.

Dans tout ce qui précède, les mesures de déplacement des palpeurs et des curseurs ont été décrites comme obtenues par des dispositifs potentiométriques. Bien entendu, on ne sortira pas du cadre de l'invention en utilisant des dispositifs magnétiques en prévoyant, par exemple, une échelle numérique magnétique sur les règles ou parties fixes et une tête de lecture magnétique en place des curseurs, avec la suppression concommittante du convertisseur analogique numérique.

L'appareil de l'invention est utilisable aussi pour déterminer objectivement le classement des carcasses de porcs.

La classification de ces carcasses se fait selon des normes définies par un catalogue communautaire reposant sur deux critères:

1. conformations,
2. épaisseur du gras.

1. La conformation est actuellement appréciée subjectivement. L'appareil permet de rendre objective cette appréciation grâce à des données mesurées.

2. L'épaisseur du gras, appelée lard chez le porc, fait déjà, dans la grille communautaire, l'objet de mensurations à l'aide d'une petite règlette à graduations millimétriques.

L'appareil permet de mesurer ce critère à l'aide d'un comparateur spécifique similaire à celui utilisé pour les mesures de développement du gigot ou du cuisseau.

Pour la mesure du jambon sur le porc, le comparateur est identique au comparateur 60 qui sert à mesurer le tende de tranche sur le boeuf et qui a été décrit plus haut en référence à la figure 5.

Pour la mesure de l'épaisseur du lard, et pour relever simultanément le profil dorsal du porc, le comparateur est identique au comparateur 70 qui a été décrit pour le boeuf en référence à la figure 6, mais ce comparateur 70 destiné au boeuf doit être complété pour le porc par un palpeur spécifique 74bis représenté en trait mixte sur la figure 6. Ce palpeur 74bis est monté sur le plateau 72 à proximité et à l'extérieur du palpeur 74. Comme ce dernier, il comprend une douille 74a fixée au plateau 72 dans laquelle est montée coulissante une tige 74b qui est pourvue du côté du palpeur 74 d'un index 74c et, de préférence, d'une poignée de manoeuvre 74d.

Toutefois, si l'état d'engraissement s'exprime par des chiffres allant de 1 à 5 pour les bovins, les veaux, les ovins et les porcs, il en va autrement pour la conformation.

Pour les bovins, les veaux et les ovins, cette dernière s'exprime officiellement par les lettres E,

U, R, O, P, et A, mais pour les porcs le code officiel est le suivant: AA, A, B et C.

En conséquence pour que l'appareil de l'invention possède un caractère universel convenant à tous types de carcasses, les chiffres ou les lettres ne seront pas gravés sur les couronnes tronconiques 101, 102, 103, mais ils seront fixés de manière détachable à la surface de ces dernières. Par exemple, on pourra prévoir des têtons de repérage et de fixation, type boutons de pression, destinés à la mise en place à l'emplacement voulu de bandes interchangeables porteuses du code approprié au type de carcasse à classer.

En outre, cette possibilité de changement et de remplacement facilite l'entretien et le nettoyage les lettres de classification.

L'emploi de l'appareil adapté au porc est le suivant:

Pour une demi-carcasse de porc, on retiendra cinq données pour déterminer la conformation:
    a) le poids,
    b) le développement musculaire du jambon,
    c) le développement de la pointe,
    d) le développement du milieu de filet,
    e) le développement du carré du filet,
et deux données pour déterminer la classe d'engraissement:
    a) l'épaisseur du lard au niveau de la pointe ou sacrum,
    b) l'épaisseur du lard au niveau du milieu de filet.

On place les deux volets 1 et 2 à 90° l'un de l'autre, comme représenté à la figure 1. On installe sur l'équerre 18 du chariot 16 un comparateur 60 (voir figure 5) par le moyen de deux tubes 61 de positionnement s'ajustant sur deux branches verticales de l'équerre 18. Les tubes 61 portent un plateau 62 disposé verticalement dans le plan de la traverse 13.

Du plateau 62 s'étendent, vers l'intérieur du dièdre formé par les volets 1 et 2, trois palpeurs du type décrit, respectivement un palpeur 63 à bout normal au dessus duquel à environ 10 cm se trouve un palpeur 64 à extrémité libre arquée horizontalement 65 et à 15 cm au dessus un autre palpeur 66 à extrémité libre arquée horizontalement 67. Les deux palpeurs en arc reproduisent sensiblement le profil superficiel du jambon.

Sur chacun des supports 24 et 34, on installe un autre comparateur 70 (voir figure 6) au moyen d'anneaux de guidage 71. Ce comparateur 70 comprend un plateau 72 installé dans le plan des potences 22 et 32 d'où s'étendent vers l'intérieur du dièdre une butée fixe 73 et deux palpeurs 74 et 74bis.

D'autre part, on pousse la barre 7 en position de butée de la demi-carcasse. On présente alors celle-ci de telle manière que sa face dorsale vienne porter contre la barre 7 et que sa face interne vienne porter contre la poutre 5, les plans verticaux le la barre 7 et de la poutre 5 définissant ainsi les plans de référence.

Le poids est le poids fiscal obtenu par une bascule.

L'unité de calcul 52 recevra l'information P de la bascule soit par une connexion calculeur-bascule, soit par une entrée manuelle à l'aide d'un clavier.

Mesure du développement du jambon (j)

Par ses mouvements dans le plan du volet 1, le chariot 16 est positionné de telle manière que le palpeur 63 vienne porter sur l'os pubien. Le mouvement est prolongé jusqu'à ce que le palpeur 67 porte contre le muscle jambon stoppant par contact le mouvement. L'enfoncement du palpeur 63 qui traduit le développement musculaire du jambon par rapport à l'os pubien est enregistré dans la mémoire.

Mesure du développement de la pointe et le l'épaisseur du lard sur ce muscle.

Par les mouvements de la potence 22 et du chariot 23, celui-ci est positionné de telle manière que le palpeur 74, par pivotement sur les paumelles 20, vienne porter contre la couenne au niveau du sacrum (point N0, fig. 7e). Le mouvement est prolongé jusqu'à ce que la butée 73 porte également sur la couenne au niveau de la pointe N1 figure 7e. L'enfoncement du palpeur 74 qui traduit le profil de la pointe est calculé par la mémoire.

L'opérateur manoeuvre le palpeur 74bis jusqu'à ce que son index 74c soit au niveau interne du lard (L1 figure 7d). L'enfoncement du papleur 74bis est calculé et enregistré par la mémoire pour être comparé à l'enfoncement du palpeur 74. La différence d'enfoncement est la mesure de l'épaisseur du lard L.

Mesure du développement du milieu de filet et de l'épaisseur du lard à ce niveau.

Par l'exécution des mouvements décrits pour la mesure de la pointe, le palpeur 74 du chariot 23 est amené à porter contre la couenne (au niveau F0 fig. 7e) de l'épine dorsale et il est enfoncé jusqu'à ce que la butée 73 porte également contre la couenne au niveau du milieu de filet (F1 fig. 7e). L'enfoncement du palpeur 74 qui traduit le développement du milieu de filet par rapport à l'épine dorsale est calculé et enregistré par la mémoire.

L'opérateur manœuvre le palpeur 74bis jusqu'à ce que son index 74c soit au niveau interne du lard (L2 fig. 7d). L'enfoncement du palpeur 74bis est calculé et enregistré par la mémoire pour être comparé à l'enfoncement du palpeur 74. La différence d'enfoncement traduit l'épaisseur du lard L.

Mesure du développement du carré de filet.

Toujours par les mouvements déjà décrits le palpeur 74 du chariot 33 est amené contre la couenne dorsale du niveau prédéterminé (M1 fig. 7e) et enfoncé jusqu'à ce que la butée 73 porte également contre la couenne au niveau du carré de filet en M2, figure 7e. L'enfoncement du palpeur 74 qui traduit le développement du carré de filet par rapport à l'épine dorsale est calculé et enregistré par la mémoire.

Les données successivement calculées par l'unité de calcul sont traitées suivant une programmation permettant d'obtenir:

1. Une note de gras s'exprimant par les chiffres 1, 2, 3.

2. Une note de conformation s'exprimant selon le catalogue communautaire par les lettres AA, A, B, C.

Le marqueur 9, quatrième élément du dispositif de l'appareil, reçoit de l'unité de calcul des impulsions électriques qui sont transformées par un moteur rotatif et il affiche le classement.

Le manipulateur saisit cet élément, encre les caractères affichés et, frappant la carcasse, marque sur cette dernière le classement affiché.

Le classement et le marquage étant effectué, l'opérateur fait pivoter les volets 2 et 3, tire sur la barre 7, ouvrant ainsi la voie pour que la carcasse soit poussée vers son lieu d'entreposage.

**Revendication**

1. Appareil pour la mesure des caractéristiques de classification et la classification des animaux de boucherie abattus, dits carcasses, comprenant un châssis (1, 2) vertical de support pour des instruments de visée montées mobiles par rapport au châssis, caractérisé en ce qu'il comprend:

a) des chariots (16, 23, 33) et/ou des curseurs (25, 26) porteurs desdits instruments de visée ainsi que d'instruments de palpage, lesdits chariots (16, 23, 33) et/ou curseurs (25, 26) étant montés coulissants en déplacement mesurable sur des potences (22, 32) et/ou traverses (13, 14) horizontales droites montées sur le châssis, réglables en hauteur mesurable et servant de guide de déplacement auxdits chariots et/ou curseurs,

b) des moyens (par exemple 27, 28) de saisie de données consistant en la mesure de déplacements mutuels et/ou par rapport au châssis d'organes de visée desdits instruments de visée et/ou d'organes de palpage (par exemple 63, 64, 66) desdits instruments de palpage,

c) des moyens (4, 5, 7) de positionnement de référence des carcasses ou demi-carcasses.

2. Appareil selon la revendication 1, caractérisé en ce que le châssis est composite et comprend au moins deux volets verticaux (1, 2) mutuellement articulés et chaque volet comprend au moins une potence (22, 32) ou traverse (13, 14) horizontale règlable en hauteur.

3. Appareil selon la revendication 1, caractérisé en ce que les moyens de saisie des données sont des moyens de mesure (27, 28, 37, 38, 47, 49) de déplacement des traverses ou potences, des chariots, de l'élongation et/ou de l'écartement des instruments de palpage, de l'écartement des instruments de visée.

4. Appareil selon la revendication 1, caractérisé en ce que les instruments de palpage et/ou de visée sont montés amovibles sur les chariots (16, 23, 33) ou curseurs (25, 26) porteurs d'instruments.

5. Appareil selon la revendication 1, caractérisé en ce qu'il comprend un calculateur programmé et éventuellement des moyens de conversion analogique-numérique des données saisies à introduire dans le calculateur et des moyens d'introduction dans le calculateur de données complémentaires telles que le poids et/ou autres indications introduites par un opérateur.

6. Appareil selon la revendication 5, caractérisé en ce qu'il comprend un dispositif d'affichage et/ou d'impression sur papier et un dispositif de marquage (9) sur les carcasses de l'indice de classification déterminé par le calculateur (52).

7. Appareil selon la revendication 1, caractérisé en ce que pour le relevé des caractéristiques tridimensionnelles des carcasses, il comporte des palpeurs à élongation mesurable lesdits palpeurs étant montés sur des comparateurs (60, 70, 75, 80).

8. Appareil selon la revendication 7, caractérisé en ce que, pour la mesure du tende de tranche, un comparateur (60) comporte au moins deux palpeurs à élongation mesurable, respectivement un palpeur (63) de positionnement sur un point défini, tel quel l'os pubien, et au moins un palpeur (64, 66) à extrémité libre arquée (65, 67) reproduisant sensiblement le profil superficiel du tende de tranche.

9. Appareil selon la revendication 7, caractérisé en ce que, pour la mesure des caractéristiques des carcasses entières, un comparateur (8) comprend un premier palpeur télescopique (82) de repérage de la séparation des noix (0), dont un élément porte une première règle (83) s'étendant perpendiculairement, sur laquelle est monté coulissant un comparateur auxiliaire (79) à trois éléments, dont deux symétriques (88) et décalés selon la direction de la règle (83) par rapport à l'élément médian (86), les éléments symétriques (88) et l'élément médian (86) constituant respectivement, l'un ou les uns, palpeur, l'autre ou les autres, butée de comparaison, pour la mesure du développement du cuisseau ou gigot.

10. Appareil selon la revendication 9, caractérisé en ce qu'un autre élément du palpeur telescopique (82) porte une seconde règle (89) s'étendant en sens opposé de la première règle (83) par rapport audit palpeur télescopique (82), pour la mesure de la fente.

11. Appareil selon la revendication 7, caractérisé en ce qu'il comprend, pour le classement des carcasses de porcs, un comparateur (70) comportant un plateau (72) sur lequel sont montés une butée fixe (73) et un premier palpeur télescopique (74), un second palpeur télescopique (74bis) étant également monté sur le plateau (72) à proximité du premier palpeur (74), ce second palpeur (74bis) comprenant une tige coulissante )74b) munie latéralement d'un index (74).

**Claims**

1. An apparatus for measurement of the classification characteristics and classification of animals slaughtered for meat, known as carcasses, comprising a vertical supporting frame (1, 2) for aiming-instruments mounted to be able to move with respect to the frame, characterized in that it comprises:

a) carriages (16, 23, 33) and/or cursors (25, 26) carrying the said aiming-instruments as well as feeler instruments, the said carriages (16, 23, 33) and/or cursors (25, 26) being mounted to be able to slide with a measurable displacement along straight horizontal brackets (22, 32) and/or crossbars (13, 14) mounted upon the frame to be adjustable to a measurable height and serving as guides for the displacement of the said carriages and/or cursors;

b) means (for example, 27, 28) of pick-up of data consisting in the measurement of displacements mutually and/or with respect to the frame, of aiming members of the said aiming instruments and/or of feeler members (for example, 63, 64, 66) of the said feeler instruments;

c) means (4, 5, 7) of reference-positioning of the carcases or half-carcases.

2. An apparatus as in Claim 1, characterized in that the frame is composite and comprises at least two vertical flaps (1, 2) hinged together and each flap comprises at least one horizontal bracket (22, 32) or crossbar (13, 14) adjustable for height.

3. An apparatus as in Claim 1, characterized in that the means of pick-up of the data are means of measurement (27, 28, 37, 38, 47, 49) of the displacement of the crossbars or brackets or carriages, of the elongation and/or of the distance apart of the feeler instruments and of the distance apart of the aiming instruments.

4. An apparatus as in Claim 1, characterized in that the feeler and/or aiming instruments are mounted detachably on the carriages (16, 23, 33) or cursors (25, 26) carrying the instruments.

5. An apparatus as in Claim 1, characterized in that it comprises a programmed calculator and possibly means of analogue-digital conversion of the data picked up for introduction into the calculator, and means of introduction into the calculator of complementary data such as the weight and/or other information indtroduced by an operator.

6. An apparatus as in Claim 5, characterized in that it comprises a device for display and/or for printing on paper, and a device (9) for marking on the carcases the classification symbol determined by the calculator (52).

7. An apparatus as in Claim 1, characterized in that for the summary of the three-dimensional characteristics of the carcases it includes feelers of measurable elongation, the said feelers being mounted on comparators (60, 70, 75, 80).

8. An apparatus as in Claim 7, characterized in that for measurement of the topside one comparator (60) includes at least two feelers of measurable elongation, being respectively one feeler (63) for positioning over a definite point such as the pubic bone, and at least one feeler (64, 66) having an arched free end (65, 67) which substantially reproduces the surface profile of the topside.

9. An apparatus as in Claim 7, characterized in that for the measurement of the characteristics of whole carcases, one comparator (80) comprises a first telescopic feeler (82) for setting the separation of the cushions (0), one element of which carries a first rule (83) which extends perpendicularly and upon which there is mounted to be able to slide an auxiliary comparator (79) having three elements, two of them being symmetrical (88) and offset along the direction of the rule (83) with respect to the central element (86), the pair of symmetrical elements (88) and the central element (86) forming respectively one the feeler and the other a stop for comparison, for the measurement of the development of the leg of veal or mutton.

10. An apparatus as in Claim 9, characterized in that another element of the telescopic feeler (82) carries a second rule (89) which extends in the direction opposite to that of the first rule (83) with respect to the said telescopic feeler (82), for measurement of the split.

An apparatus as in Claim 7, characterized in that it comprises for the classification of the carcases of pigs a comparator (70) including a plate (72) upon which are mounted a fixed stop (73) and a first telescopic feeler (74), a second telescopic feeler (74 bis) being likewise mounted on the plate (72) close to the first feeler (74), this second feeler (74 bis) comprising a sliding rod (74b) equipped at the side with a pointer (74c).

## Patentansprüche

1. Apparat zum Messen der Klassifikationscharakteristika und der Klassifikation von getöteten Schlachttieren, genannt Tierkörper, umfassend einen vertikalen Tragrahmen (1, 2) für Beobachtungsinstrumente, die gegenüber dem Rahmen beweglich montiert sind, dadurch gekennzeichnet, dass er umfasst:

a) Wagen (16, 23, 33) und/oder Läufer (25, 26) als Träger der erwähnten Beobachtungsinstrumente und auch von Abtastinstrumenten, wobei die Wagen (16, 23, 33) und/oder Läufer (25, 26) mit messbarer Verschiebung auf Armen (22, 23) und/oder Querbalken (13, 14) gleitend montiert sind, die am Rahmen gerade montiert sind und der Höhe nach messbar verstellbar sind und als Verstellführung für die erwähnten Wagen und/oder Läufer dienen,

b) Einrichtungen (z.B. 27, 28) zum Erfassen von Daten, bestehend im Mass der gegenseitigen Verschiebungen und/oder gegenüber dem Chassis der erwähnten Beobachtungsorgange der Beobachtungsinstrumente und/oder der Abtastorgane (beispielsweise 63, 64, 66, der erwähnten Abtastinstrumente,

c) Einrichtungen (4, 5, 7) zum Positionieren von Tierkörpern oder Halbtierkörpern in Bezugslage.

2. Apparat nach Anspruch 1, dadurch gekennzeichnet, dass der Rahmen zusammengesetzt ist und wenigstens zwei vertikale Flügel (1, 2) umfasst, die gegenseitig gelenkig gelagert sind und jeder Flügel wenigstens einen horizontalen, der Höhe nach verstellbaren Arm (22, 32) oder Querbalken (13, 14) umfasst.

3. Apparat nach Anspruch 1, dadurch gekennzeichnet, dass die Einrichtungen zum Erfassen von Daten Einrichtungen zum Messen (27, 28, 37, 38, 47, 49) der Verschiebungen der Querbalken oder Arme, der Wagen, der Ausdehnung und/oder des Abstandes von Abtastinstrumenten, des Abstandes von Beobachtungsinstrumenten sind.

4. Apparat nach Anspruch 1, dadurch gekennzeichnet, dass die Abtast- und/oder Beobachtungsinstrumente auf den Wagen (16, 23, 33) oder Läufern (25, 26) als Träger der Instrumente lösbar sind.

5. Apparat nach Anspruch 1, dadurch gekennzeichnet, dass er einen programmierten Rechner und gegebenenfalls Analog-Digital-Wandler für die erfassten, in den Rechner einzugebenden Daten aufweist und Einrichtungen zum Eingeben von ergänzenden Daten, wie das Gewicht und/oder andere durch eine Bedienungsperson eingegebene Angaben in den Rechner.

6. Apparat nach Anspruch 5, dadurch gekennzeichnet, dass er ein Anzeigegerät und/oder einen Papierdrucker und ein Gerät zum Markieren (9) des vom Rechner (52) bestimmten Klassifikationsindex auf den Tierkörpern aufweist.

7. Apparat nach Anspruch 1, dadurch gekennzeichnet, dass er zum Ermitteln dreidimensionaler Charakteristika der Tierkörper Abtaster mit messbarer Ausziehweite besitzt, wobei diese Abtaster auf Komparatoren (60, 70, 75, 80) montiert sind.

8. Gerät nach Anspruch 7, dadurch gekennzeichnet, dass für die Messung der Richtung des Schnittes ein Komparator (60) wenigstens zwei Abtaster mit messbarer Ausziehweite umfasst, und zwar einen Abtaster (63) für die Anlage an einem definierten Punkt, wie dem Schambein, und wenigstens einen Abtaster (64, 66) mit gebogenem freiem Ende (65, 67), der im wesentlichen das Hüllprofil der Richtung des Schnittes wiedergibt.

9. Apparat nach Anspruch 7, dadurch gekennzeichnet, dass zur Messung von Charakteristika ganzer Tierkörper ein Komparator (80) einen ersten teleskopierbaren Abtaster (82) für das Anpeilen der Trennung der Nüsse (0) umfasst, von dem ein Element eine erste Schiene (83) trägt, die sich senkrecht erstreckt, auf welcher gleitend ein Hilfskomparator (79) mit drei Elementen befestigt ist, von welchen zwei symmetrisch (88) und in Richtung der Schiene (83) gegenüber dem mittleren Element (86) versetzt sind, wobei die symmetrischen Elemente (8) und das mittlere Element (86) jeweils den einen oder die einen Abtaster, das andere oder die anderen Vergleichsanschläge für die Messung der Entwicklung des Lendenstückes oder der Keule bilden.

10. Apparat nach Anspruch 9, dadurch gekennzeichnet, dass ein anderes Element des teleskopierbaren Abtasters (82) eine zweite Schiene (89) trägt, die sich für die Messung der Spalte, bezogen auf den teleskopierbaren Taster (82) in entgegengesetzter Richtung zur ersten Schiene (83) erstreckt.

Fig:1

*Fig.2*

*Fig.3*

Fig.4

0046704

Fig:5

67

66

65

64

63

60

61

62

61

L

74d

74c

74b

Fig:6

73

70

74

72

74 bis

71

72

74a

17

Fig:7

Fig:7a            Fig:7b            Fig:7c

Fig. 7d

Fig. 7e

*Fig. 8*

*Fig. 9*

0046704

Fig. 10

25